(19) Europäisches Patentamt / European Patent Office / Office européen des brevets

(11) **EP 4 263 676 B1**

(12) **EUROPEAN PATENT SPECIFICATION**

(45) Date of publication and mention of the grant of the patent:
**30.10.2024 Bulletin 2024/44**

(21) Application number: **21840384.8**

(22) Date of filing: **17.12.2021**

(51) International Patent Classification (IPC):
**C08H 7/00** $^{(2011.01)}$    **C07D 307/50** $^{(2006.01)}$

(52) Cooperative Patent Classification (CPC):
**C07D 307/50**

(86) International application number:
**PCT/US2021/064077**

(87) International publication number:
**WO 2022/133243 (23.06.2022 Gazette 2022/25)**

(54) **METHODS AND SYSTEMS FOR PRODUCTION OF FURFURAL**

VERFAHREN UND SYSTEME ZUR HERSTELLUNG VON FURFURAL

PROCÉDÉS ET SYSTÈMES DE PRODUCTION DE FURFURAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.12.2020 EP 20215448**
**12.08.2021 US 202117400772**

(43) Date of publication of application:
**25.10.2023 Bulletin 2023/43**

(73) Proprietor: **Shell Internationale Research Maatschappij B.V.**
**2596 HR The Hague (NL)**

(72) Inventors:
• **LANGE, Jean Paul Andre Marie Joseph Ghislain**
**1031 HW Amsterdam (NL)**
• **RICCIARDI, Luca**
**7522 NB Enschede (NL)**
• **VERBOOM, Willem**
**7522 NB Enschede (NL)**
• **HÜSKENS, Jurriaan**
**7522 NB Enschede (NL)**
• **CHHEDA, Juben Nemchand**
**Houston, Texas 77082-3101 (US)**

(74) Representative: **Shell Legal Services IP**
**PO Box 384**
**2501 CJ The Hague (NL)**

(56) References cited:
**WO-A1-2015/066287**

• **RICCIARDI LUCA: "Furfural Manufacture from Xylose: Routes towards High Selectivity and Novel Processes", 29 October 2021 (2021-10-29), pages 1 - 161, XP055897189, Retrieved from the Internet <URL:https://research.utwente.nl/files/265609691/Thesis_Luca_Ricciardi_2021.pdf> [retrieved on 20220303]**

**Description**

**FIELD OF THE INVENTION**

The present invention relates to methods and systems for production of furfural from a xylose-containing solution.

**BACKGROUND OF THE INVENTION**

Furfural plays an important role in the chemical industry as a precursor of furan and derivatives of furan, including furfuryl alcohol. Also, furfural is used for the production of resins by condensation reaction of furfural with formaldehyde, phenol, acetone or urea. In addition, furfural can be used as a solvent, vulcanization enhancer, insecticide, fungicide, germicide, or in the production of such compounds, as well as for use as a potential fuel.

Furfural is an attractive compound because it can be produced from renewable resources. One potential source of renewable (non-fossil) feedstock for the production of furfural are substances selected from the group consisting of xylose, oligosaccharides comprising xylose units and polysaccharides comprising xylose units originating from cellulose-containing biomass.

Xylose is a monosaccharide also referred to as wood sugar which belongs to the group of pentoses. Oligo- and polysaccharides which comprise xylose units typically occur in plants, especially in woody parts of plants, in straw, and in the seeds or the shells of the seeds of several plants. Oligo- and polysaccharides which consist of xylose units are generally referred to as xylans. Oligo- and polysaccharides which consist of xylose units and other monosaccharide units are generally referred to as heteroxylans. Xylans and heteroxylans belong to the group of polyoses. Polyoses (earlier also referred to as hemicellulose) are polysaccharides which in plant biomass typically occur in a composite wherein said polyoses and lignin are incorporated between cellulose fibres. Dry plant biomass (water content below 15 wt.-%) which comprises cellulose, polyoses and lignin is also referred to hereinabove and hereinbelow as lignocellulose.

One general process to produce furfural from xylose in biomass material is "aqueous dehydration" using batchwise or continuous acid-catalysed dehydration. This type of aqueous dehydration process provides a yield of about 30-50 mol% furfural (meaning only 30 - 50 % of the total moles of xylose is converted to furfural) (Furfural - a promising platform for lignocellulosic biofuels by J.-P. Lange, E. van der Heide, J. van Buijtenen, R.J. Price, ChemSusChem 2012, 5, 150-166). With such low yields, it degrades 50-70 mol% of the valuable xylose into undesirable by-products that foul equipment and contaminate the water stream.

Another process which has improved yields over aqueous dehydration is "biphasic dehydration," which adds a water-insoluble solution to the aqueous dehydration to extract the furfural into an organic phase to protect it from further degradation, and optionally a salt in the aqueous phase to further assist the extraction of furfural into the water-insoluble solution (Lange et al. 2012). While biphasic dehydration can increase the yield to 60 to 70 mol%, it still degrades 30 to 40 mol% of the valuable xylose into undesirable by-products that foul equipment and build up in the solvent recycle stream.

Yet another process with improved yields over biphasic dehydration is to extract and recover xylose as a solid product from hydrolysis of biomass and subjecting the recovered xylose in a dehydration reaction (B. R. Caes, R. T. Raines, ChemSusChem 2011, 4, 353 - 356; L. Shuai, J. Luterbacher, ChemSusChem 2016, 9,133-155). Because the xylose is not in solution, the dehydration reaction can be carried out in a single phase under conditions favourable to the furfural conversion, such as using polar aprotic solvents which can provide furfural yields of 90 mol%. However, this process requires isolation of the xylose from the hydrolysate, e.g., by distilling out all the water, which can be highly energy demanding. Such isolation process to recover the xylose in solid form can further concentrate contaminants of the xylose streams in the solid xylose end product.

Although there are processes to provide improved isolation xylose from hydrolysate, such as (US10407453), these processes nonetheless, are directed to providing the xylose in dry form to allow the xylose to then be converted and/or used in the production of a C5 sugar-platform of biochemical and biofuels.

WO 2015/066287 A1 discloses a method for producing furfural, comprising: enzymatically isomerizing xylose to xylulose in an aqueous solution; extracting both xylose and xylulose with an immiscible non-aqueous phase containing a water-insoluble boronic acid (such as a phenyl boronic acid or a naphthalene boronic acid) which forms diboronate esters with the two isomers and drives the isomerisation reaction towards formation of more xylulose; extracting xylulose from the immiscible non-aqueous phase with a mixture of acidic aqueous and aprotic solvents such as DMSO; heating the mixture from previous step, containing extracted xylulose, to dehydrate xylulose to furfural.

It would, therefore, be advantageous to provide a process for the production of furfural from a xylose-containing aqueous solution with a relatively higher yield without expensive isolation of xylose in dry form.

## SUMMARY OF THE INVENTION

Accordingly, the present invention provides a process for a method for producing furfural comprising: (a) providing an aqueous xylose-containing solution comprising xylose in an amount of greater than or equal to 0.5 wt.%, (b) providing an extraction solution comprising a water-insoluble boronic acid (BA: $R-B(OH)_2$) and a water-insoluble solvent; and (c) combining the xylose-containing solution with the extraction solution to provide a first combined solution. The ratio of boronic acid to xylose in the first combined solution is greater than 1:1 molar, respectively, and the first combined solution comprises a first aqueous phase and a first non-aqueous phase which comprises at least a portion of the xylose as xylose-diboronate ester ($BA_2X$). The method further comprises (d) separating at least a portion (preferably a majority of greater than 50%, more preferably greater than 75%, and most preferably greater than 90%) of the first non-aqueous phase from the first combined solution; and (e) providing an ionic conversion solution having a pH of less than or equal to 4. The ionic conversion solution comprises one or more salts with each salt comprises a plurality of ions selected from the group consisting of an anion, a cation, and a combination thereof, and the conversion solution has a calculated molar ionic strength of at least 1, preferably at least 2, more preferably at least 3, and most preferably at least 4. The calculated molar ionic strength is determined according to equation (1):

$$I = \tfrac{1}{2} \text{sum}(c_i * z_i^2) \qquad (1)$$

where

I is the calculated molar ionic strength;
i is an ion of the salt(s)
$c_i$ is the molar concentration of ion i (M, mol/L) in the ionic conversion solution, and
$z_i$ is the charge number of ion i
and the summation is over ions from the one or more salts.

The method further comprises (f) combining at least a portion of the first non-aqueous phase with the ionic conversion solution in a ratio of conversion solution to the non-aqueous phase in a range from 0.1 and up to 10.0 by volume, respectively, preferably from 0.3 and up to 3 by volume, and more preferably from 0.5 and up to 1, to form a second combined solution. The method further comprises (g) providing the second combined solution with a reaction temperature of at least 130 °C, preferably at least 150 °C, more preferably at least 170 °C, and most preferably at least 200 °C to form heated second combined solution to convert at least a portion of the xylose-diboronate ester into furfural. The heated second combined solution comprises

a second aqueous phase comprising water and at least 90% of the ions in the second combined solution, and
a second non-aqueous phase comprising greater than 50% (preferably greater than 75% and most preferably greater than 90%) of the water-insoluble solvent, greater than 50% (preferably greater than 75%) of the water-insoluble boronic acid, and at least 50% of the furfural in the second combined solution. The method further comprises (h) separating at least a portion of the second non-aqueous phase from the second combined solution; and (i) recovering at least a portion of the furfural from the second non-aqueous phase.

Optionally, the salt is an organic salt, or an inorganic salt, or a combination thereof, wherein the salt is water soluble and solvent insoluble at temperature in a range of 20 °C to 200 ° C. Optionally, a combination of the salt and the acid are selected from the group consisting of (i) $Na_2SO_4$ or $MgSO_4$ with $H_2SO_4$, and(ii) NaCl or MgCh with HCl.
Optionally, the pH of the conversion solution is provided by using at least an acid selected from the group consisting of an organic acid, an inorganic acid, and a combination thereof. Optionally, the acid is an inorganic acid selected from the group consisting of HCl, $H_2SO_4$, $H_3PO_4$, and any combination thereof.
Optionally, step (g) of providing the second combined solution with the reaction temperature comprises heating the first non-aqueous phase to the reaction temperature; heating the ionic conversion solution to the reaction temperature; and combining the heated first non-aqueous phase with the heated ionic conversion solution to form the heated second combined solution.
Optionally, when the first aqueous phase comprises water-insoluble solvent, and water-insoluble boronic acid, the method further comprises further processing at least a portion (preferably a majority of greater than 50%, more preferably greater than 75%, and most preferably greater than 90%) of the separated first aqueous phase to recover at least a portion of the water-insoluble solvent, or the water-insoluble boronic acid, or a combination thereof.
Optionally, the method further comprises performing at least a portion of steps (c) and (d) in a liquid-liquid extraction unit in counter-current operation, wherein the xylose-containing solution is provided at a higher temperature than the temperature of the extraction solution.

Optionally, the xylose-containing solution is a hydrolysate.

Optionally, at least one of the water-insoluble boronic acid and the water-insoluble solvent has up to 5 wt.% solubility in water at 20 °C. Optionally, at least one of the water-insoluble boronic acid and water-insoluble solvent has a boiling point higher than that of furfural, preferably at least 2 °C higher.

Optionally, the water-insoluble boronic acid is selected from the group consisting of phenylboronic acid, 4-biphenylboronic acid, 4-butylphenyl boronic acid, 4-tert-Butylphenyl boronic acid, 4-ethylphenyl boronic acid, 2-naphthylboronic acid, naphthalene-1-boronic acid, o-tolylboronic acid, m-tolylboronic acid, (2-methylpropyl) boronic acid, butylboronic acid, octylboronic acid, phenethyl boronic acid, cyclohexyl boronic acid, and any combination thereof.

Optionally, the water-insoluble solvent is selected from the group consisting of benzoic acid, cresol (m), di-isopropyl ether, terephthalic acid, diethylene glycol diethyl ether, anisole, salicylic acid, 2,6 xylenol, 4Et-phenol, toluene, benzofuran, ethylbenzene, octanoic acid, 1-methylnaphtalene, nitrobenzene, guaiacol, heptane, 1-octanol, and methyl isobutyl ketones, an any combination thereof.

Optionally, step (i) comprises providing at least a portion of the second non-aqueous phase from (h) to a distillation process to recover an overhead product comprising furfural and a bottom product comprising water-insoluble solvent and water-insoluble boronic acid.

Optionally, the method further comprises providing at least a portion (preferably a majority of greater than 50%, more preferably greater than 75%, and most preferably greater than 90%) of the bottom product for use as part of the extraction solution. Optionally, the method further comprises providing at least a portion (preferably a majority of greater than 50%, more preferably greater than 75%, and most preferably greater than 90%) of the second aqueous phase for use as part of the ionic conversion solution.

## BRIEF DESCRIPTION OF THE DRAWINGS

Fig. 1 illustrates one embodiment of the processes to produce furfural according to certain aspects disclosed herein.
Fig. 2 illustrates one embodiment of the systems to produce furfural according to certain aspects disclosed herein.
Fig. 3 is a graph of Table 4 in Example 2.
Fig. 4 is a graph of Table 5 in Example 3.

## DETAILED DESCRIPTION OF THE INVENTION

The present invention will now be described in detail with reference to embodiments thereof as illustrated in the accompanying drawings. References to "one embodiment", "an embodiment" "an example embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. Other suitable modifications and adaptations of the variety of conditions and parameters normally encountered in the field, and which would be apparent to those skilled in the art, are within the spirit and scope of the invention.

Although the description herein provides numerous specific details that are set forth for a thorough understanding of illustrative embodiments, it will be apparent to one skilled in the art that embodiments may be practiced without some or all of these specific details. In other instances, well known process steps and/or structures have not been described in detail in order to not unnecessarily obscure the present invention. The features and advantages of embodiments may be better understood with reference to the drawings and discussions that follow.

In addition, when like elements are used in one or more figures, identical reference characters will be used in each figure, and a detailed description of the element will be provided only at its first occurrence. Some features or components of the systems or processes described herein may be omitted in certain depicted configurations in the interest of clarity. Moreover, certain features such as, but not limited to pumps, valves, gas bleeds, gas inlets, fluid inlets, fluid outlets and the like have not necessarily been depicted in the figures, but their presence and function will be understood by one having ordinary skill in the art. Similarly, the depiction of some of such features in the figures does not indicate that all of them are depicted.

The present inventors have surprisingly found that xylose in an aqueous solution can be extracted as xylose-diboronate ester (BA$_2$X) into the non-aqueous phase of an extraction solution comprising a water-insoluble solvent and a water-insoluble boronic acid, and the non-aqueous phase can be separated for conversion (or dehydration) of the xylose-diboronate ester (BA$_2$X) into furfural upon contacting with an ionic conversion solution having a pH of less than or equal to 4, said ionic conversion solution comprising one or more salts, wherein each salt comprises a plurality of ions selected from the group consisting of an anion, a cation, and a combination thereof. The ionic conversion solution has a calculated molar ionic strength of at least 1. The calculated molar ionic strength is determined according to equation (1):

$$I = ½ \ \text{sum}(c_i * z_i^2) \qquad (1)$$

wherein

I is the calculated molar ionic strength;
i is an ion of the salt(s)
$c_i$ is the molar concentration of ion i (M, mol/L) in the ionic conversion solution, and
$z_i$ is the charge number of ion i

and the summation is over ions from the one or more salts.

The reaction mixture is then separated into a water-insoluble phase, which contain furfural, and the ionic conversion solution. The furfural can then be recovered from the water-insoluble phase using any suitable methods.

As used herein, "aqueous solution" has its ordinary meaning, which is a solution in which a solute is dissolved in a solvent, and the solvent is water. "Water-insoluble" also has its ordinary meaning, which describes the low solubility of a substance in water. Low solubility means preferably up to 5 wt.% solubility at 20 °C, including up to 2 wt.% solubility, up to 1 wt.% solubility, up to 0.5 wt.% solubility, or up to 0.1 wt.% solubility. Alternatively, "water-insoluble" as used herein describes a substance with an octanol-water partition coefficient LogP (also called LogKow) of at least 1, including at least 2, or at least 3. "Water-soluble" as used herein describes a substance with an octanol-water partition coefficient LogP of at most 0, preferably at most (-0.5), more preferably at most (-1.0). "Aqueous phase" has its ordinary meaning, which describes a liquid phase in which the concentration of water is greater than the concentration of water-insoluble liquid component(s). "Non-aqueous" has its ordinary meaning, which describes a liquid phase in which the concentration of water-insoluble liquid component(s) is greater than the concentration of water.

Accordingly, the present disclosure provides for a process for producing furfural comprising:

a. providing a xylose-containing solution comprising xylose in an amount of greater than or equal to 0.5 wt.%, wherein the xylose-containing solution is an aqueous solution;
b. providing an extraction solution comprising a water-insoluble boronic acid (BA: R-B(OH)$_2$) and a water-insoluble solvent;
c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester (BA$_2$X);
d. separating at least a portion of the first non-aqueous phase from the first combined solution;
e. providing an ionic conversion solution having a pH of less than or equal to 4, said ionic conversion solution comprising one or more salts, wherein each salt comprises a plurality of ions selected from the group consisting of an anion, a cation, and a combination thereof, wherein the ionic conversion solution has a calculated molar ionic strength of at least 1, and wherein the calculated molar ionic strength is determined according to equation (1):

$$I = ½ \ \text{sum}(c_i * z_i^2) \qquad (1)$$

wherein

I is the calculated molar ionic strength;
i is an ion of the salt(s)
$c_i$ is the molar concentration of ion i (M, mol/L) in the conversion solution, and
$z_i$ is the charge number of ion i
and the summation is over ions from the one or more salts;

f. combining at least a portion of the non-aqueous phase from (d) with the ionic conversion solution from (e) in a ratio of conversion solution to the non-aqueous phase in a range from 0.1 and up to 10.0 by volume, respectively, preferably from 0.3 and up to 3 by volume, and more preferably from 0.5 and up to 1, to form a second combined solution;

g. heating the second combined solution to at least 130 degrees C to convert at least a portion of the xylose-diboronate ester into furfural, wherein the heated second combined solution comprises

a second aqueous phase comprising water and at least 90% of the ions in the second combined solution, and a second non-aqueous phase comprising greater than 50% of the water-insoluble solvent, greater than 50% of the water-insoluble boronic acid, and at least 50% of the furfural in the second combined solution;

h. separating at least a portion of the second non-aqueous phase from the second combined solution; and

i. recovering at least a portion of the furfural from the second non-aqueous phase.

## Xylose-containing solution

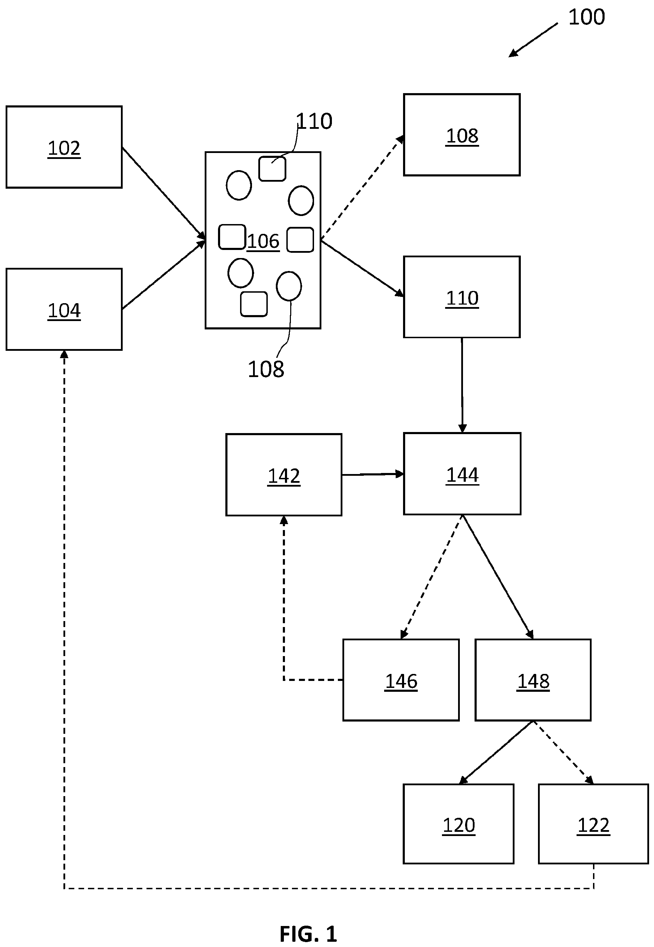

Referring to FIG. 1, the process comprises providing a xylose-containing aqueous solution 102 comprising xylose in an amount of greater than or equal to 0.5 wt.%, including preferably greater than or equal to 1.0 wt.%, more preferably greater than or equal to 2.0 wt.%, or most preferably greater than or equal to 3.0 wt.%.

The process described herein can be suitable for use with a xylose-containing aqueous solution with any pH, from 1 to 14. That is, the process described herein can be used with xylose-containing aqueous solution 102 that is acidic with a pH in a range from 1 to 6, xylose-containing aqueous solution 102 that is basic with a pH in a range of 8 to 14, or xylose-containing aqueous solution 102 that is neutral with a pH from greater than 6 to less than 8.

While any xylose-containing solution as described herein may be provided for use in the process, a suitable xylose-containing solution 102 can include one that is derived from a pre-treatment step in which a cellulosic biomass is hydrolysed by methods known by one of ordinary skill in the art, including hot water at neutral pH (e.g. steam explosion), hot water at acidic pH e.g. by addition of organic or inorganic acids (e.g. dilute acid and reversible-acid pre-treatment), or hot water at basic pH e.g. by addition of organic or inorganic base (e.g. kraft pulping), as described e.g. by Steinbach, Kruse, Sauer, Biomass Conv. Bioref. (2017) 7:247-274, as well as those methods that employ ionic liquids.

In a preferred embodiment the term "cellulosic biomass" refers to biomass comprising a) cellulose as well as b) one or more substances selected from the group consisting of polyoses and other sources of xylose units. For example, lignocellulose is cellulosic biomass that can serve as a source of xylose units. Suitable cellulosic biomass, particularly lignocellulose, includes any material and/or agricultural biomass having a hemicellulose concentration of at least 10%, at least 15%, at least 20%, at least 25%, at least 30%, or at least 50%. Exemplary lignocellulosic biomasses that can be used in this regard include, but are not limited to: corn cobs, crop residues such as corn husks, corn stover, grasses, wheat, wheat straw, barley straw, hay, rice straw, switchgrass, waste paper, sugar cane bagasse, sorghum, components obtained from milling of grains, trees, branches, roots, leaves, wood chips, sawdust, shrubs and bushes, vegetables, fruits, flowers and animal manure, soy hulls from soybean processing, rice hulls from rice milling, corn fibre from wet milling or dry milling, bagasse from sugarcane processing, pulp from sugar beets processing, distillers grains, and the like.

Suitably, the pre-treatment step as described in WO2016025678 and WO2016025679 can be used to hydrolyze cellulosic biomass to produce a xylose-containing solution that may be used in the process described herein. As noted above, such product of hydrolysis may be referred to as a hydrolysate, which comprises xylose in an amount of at least 0.5 wt.%, including preferably at least 1.0 wt.%, at least 2.0 wt.%, or most preferably at least 3.0 wt.%.

Preferably, xylose-containing aqueous solution 102 may be a product of an acidic pre-treatment based on diluted $H_2SO_4$ or concentrated $\alpha$-hydroxyethane sulfonic acid (HESA) as acid, as described in U.S. Patent No. 9290821, the content of which is incorporated by reference in its entirety.

Xylose-containing solution 102 is an aqueous solution, which means it is a solution in which the solvent is liquid water.

## Extraction solution

Referring to FIG. 1, the process further comprises providing an extraction solution 104 comprising an organic or water-insoluble boronic acid (BA: R-B(OH)$_2$) and a water-insoluble solvent.

A suitable water-insoluble boronic acid is one with low water solubility, preferably up to 5 wt.% solubility (meaning the selected water-insoluble boronic acid is soluble up to 5 wt.% in water at 20°C), including up to 2 wt.% solubility, up to 1 wt.% solubility, up to 0.5 wt.% solubility, or up to 0.1 wt.% solubility. Alternatively, a suitable water-insoluble boronic acid is one with an octanol-water partition coefficient LogP (also called LogKow) of at least 1, including at least 2, or at least 3.

In a preferred embodiment, a suitable water-insoluble boronic acid has an atmospheric boiling point that is higher than the atmospheric boiling point (Tb) of furfural, which is 162°C, to allow for use of distillation as an option for recovery of furfural as an overhead product. Preferably, the suitable water-insoluble boronic acid has an atmospheric boiling point that is at least 2 °C higher (i.e., atmospheric boiling point of at least 164°C), more preferably at least 5 °C (i.e., atmospheric boiling point of at least 167°C).

For instance, one exemplary suitable water-insoluble boronic acid is phenyl boronic acid, which has a water solubility of 1 wt.% at 20°C and a logP of 1.59 and an atmospheric boiling point Tb of 265°C. Other substituted phenyl boronate compounds are also suitable, such as alkyl phenyl boronate, naphthanyl boronate and substituted naphthanyl boronate, and any combination thereof. For example, one suitable example is 2-napthyl boronic acid, which has a water solubility of 0.03 wt.% at 20°C and a logP of 2.82 and a Tb of 382°C. Another example is octylboronic acid which is reported as water-insoluble and has a logP of 3.56 and a Tb of 262°C.

Table 1 below shows examples of suitable water-insoluble boronic acid that can be used in extraction solution 104, along with their solubility description and/or LogP, and atmospheric boiling point (Tb).

**TABLE 1**

| Name | Water solubility g/L (20°C) | LogP | Tb °C |
|---|---|---|---|
| phenylboronic acid | 10 | 1.59 | 265 |
| 4-biphenylboronic acid | Insoluble | 3.35 | |
| 4-butylphenyl boronic acid | Insoluble | 3.64 | |
| 4-tert-Butylphenyl boronic acid | Insoluble | 3.28 | |
| 4-ethylphenyl boronic acid | N.A. | 2.58 | |
| 2-naphthylboronic acid | 0.3 | 2.82 | 382 |
| naphthalene-1-boronic acid | 0.3 | | |
| o-tolylboronic acid | Slightly soluble | 2.05 | 283 |
| m-tolvlboronic acid | 25 | 2.05 | 290 |
| (2-methylpropyl) boronic acid | 33 | 0.6 | 180 |
| butvlboronic acid | 25 | 1.43 | 189 |
| octylboronic acid | Insoluble | 3.56 | 262 |
| Phenethyl boronic acid | 1.8 | 1.20 | |
| Cvclohexvl boronic acid | 25 | 1.90 | 253 |

One or more (such as two or more) suitable water-insoluble boronic acid as described here can be used in extraction solution 104 as described herein based on design choices by one of ordinary skill in the art. While certain descriptions may refer to "a water-insoluble boronic acid," "the water-insoluble boronic acid," or "the boronic acid," it is understood that such reference can include more than one (such as two or more) water-insoluble boronic acids, as applicable.

Referring to FIG. 1, extraction solution 104 further comprises a water-insoluble solvent. A suitable water-insoluble solvent is one with low water solubility to lower the likelihood of dissolution in water, preferably up to 5 wt.% solubility (meaning the selected water-insoluble solvent is soluble up to 5 wt.% of in water at 20°C), including up to 2 wt.% solubility, up to 1 wt.% solubility, up to 0.5 wt.% solubility, or up to 0.1 wt.% solubility. Alternatively, a suitable water-insoluble solvent is one with an octanol-water partition coefficient LogP (also called LogKow) of at least 1, including at least 2, or at least 3 and up to 7, up to 6, or up to 5, preferably from 1 to 7, more preferably from 2 to 6, and most preferably from 3 to 5.

In a preferred embodiment, the suitable water-insoluble solvent or the water-insoluble boronic acid, and most preferably, both, has an atmospheric boiling point that is higher than the atmospheric boiling point of furfural, which is 162°C, to allow for use of distillation as an option for recovery of furfural as an overhead product. Preferably, the suitable water-insoluble boronic acid or the suitable water-insoluble solvent, and most preferably, both, has an atmospheric boiling point that is at least 2 °C higher (i.e., atmospheric boiling point of at least 164°C), more preferably at least 5 °C (i.e., atmospheric boiling point of at least 167°C).

In a preferred embodiment, the water-insoluble solvent has a good affinity for the xylose-diboronate ester and a good affinity for furfural. By affinity we mean a high partition coefficient of the $BA_2X$ or furfural between extraction solution 104 and water in first combined solution 106 (described further below), including such partition coefficient of at least 0.1, preferably at least 0.5, preferably at least 1.0, and most preferably at least 2.

Examples of suitable water-insoluble solvent that have good affinity for the xylose-diboronate ester and a good affinity for furfural include aromatic hydrocarbons, preferably toluene and most preferably methyl naphthalene or aromatic mixtures rich in alkylbenzene or alkyl-naphthalene components. Water-insoluble solvent also include aromatic components that carry heteroatoms such as nitrobenzene, anisole, guaiacol, cresols, as well as aliphatic components free of heteroatoms (e.g., heptane and other alkanes) or containing heteroatoms (e.g., 1-octanol, methyl isobutyl ketones)

Table 2 below shows examples of suitable water-insoluble solvents that can be used in extraction solution 104, along with their solubility (LogP) and atmospheric boiling point (Tb).

**TABLE 2**

| Name | Water solubility g/L (20 °C) | LogP | Tb °C |
|---|---|---|---|
| guaiacol | 18.7 (25°C) | 0.97 | 205 |
| methyl isobutyl ketone | 19 | 1.25 | 117 |
| nitrobenzene | 2.1 (25°C) | 1.85 | 210 |
| Benzoic acid | 3.4 | 1.87 | 249 |
| Cresol (m) | 23 | 1.94 | 203 |
| Diisopropyl ether | 1.7 | 2.03 | 68 |
| terephthalic acid | 0.015 | 2.00 | 392 |
| anisole | 1.5 | 2.11 | 153 |
| Salicylic acid | 2.0 | 2.26 | high |
| 2,6 xylenol | 6.2 | 2.40 | 201 |
| 4Et-phenol | 6.1 | 2.47 | 218 |
| Toluene | 0.52 (25°C) | 2.50 | 111 |
| Benzofuran | insoluble | 2.67 | 174 |
| Ethylbenzene | 0.15 | 2.96 | 136 |
| 1-octanol | 0.3 | 3.10 | 195 |
| octanoic acid | 0.9 | 3.32 | 237 |
| 1-methylnaphtalene | 0.026 | 3.63 | 241 |
| heptane | 0.003 | 4.47 | 98 |

One or more (such as two or more) suitable water-insoluble solvents as described here can be used in extraction solution 104 as described herein based on design choices by one of ordinary skill in the art. While certain descriptions may refer to "a water-insoluble solvent" or "the water-insoluble solvent," it is understood that such reference can include more than one (such as two or more) water-insoluble solvents, as applicable.

Suitably, extraction solution 104 can comprise at least 1 wt.%, including at least 5 wt.%, at least 10 wt.%, or at least 20 wt.% of the water-insoluble boronic acid and up to 99 wt.%, including up to 95 wt.%, up to 90 wt.%, or up to 80 wt.% of the water-insoluble solvent.

**First combined solution**

Referring to FIG. 1, the process further comprises combining an amount of xylose-containing solution 102 with an amount of extraction solution 104 to provide first combined solution 106, wherein the ratio of boronic acid to xylose in first combined solution 106 is at least 1:1 molar, respectively, preferably at least 1.5:1 and most preferably at least 2:1. It is understood that one of ordinary skill would be capable of (i) determining the amount of xylose in xylose-containing solution 102 (if such amount is unknown) using methods such as high-pressure liquid chromatography (HPLC), (ii) preparing extraction solution 104 with a suitable amount of water-insoluble boronic acid and water-insoluble solvent, in accordance with the description provided herein, and (iii) determining the amount of such extraction solution 104 to be added to a particular amount of xylose-containing solution 102 so that the ratio of boronic acid to xylose in first combined solution 106 is at least 1:1 molar.

When xylose-containing solution 102 is combined with extraction solution 104 to form first combined solution 106, at least a portion of the xylose from solution 102 comes into contact with at least a portion of organic boronic acid from solution 104. This contact allows the xylose to be converted to xylose monoboronate and subsequently xylose-diboronate ester, which has low solubility in water, so it has a greater affinity toward the water-insoluble solvent of solution 104 that is in solution 106. As more xylose is converted to xylose-diboronate esters, a non-aqueous phase of first combined solution 106 (eventually non-aqueous phase 110 once separated from solution 106) comprising (i) boronic acid and water-insoluble solvent from extraction solution 104 and (ii) xylose-diboronate esters begins to form. Correspondingly, an aqueous phase of first combined solution (eventually non-aqueous phase 110 once separated from solution 106) begins to form as well, where the aqueous phase has less xylose than xylose-containing solution 102.

After an amount of time, which can be determined or selected by one of ordinary skill to achieve certain desired objectives, first combined solution 106 comprises aqueous phase 108 and a non-aqueous phase 110, said non-aqueous phase comprising at least a portion of the xylose from xylose-containing solution 102 as xylose-diboronate ester ($BA_2X$). Preferably, non-aqueous phase 110 comprises at least 20 mol% of the xylose from xylose-containing solution 102 as xylose-diboronate ester, more preferably at least 50 mol%, including at least 70 mol% or at least 90 mol%.

Preferably, first combined solution 106 is mixed using suitable methods, such as mixing, stirring, static mixer, turbulent flow, jet loop, etc to allow xylose and organic boronic acids molecules to interact, thereby improving the yield of xylose-diboronate-ester that forms. Examples of suitable methods for mixing can additionally or alternatively include internal components of the separation or extraction units noted above. Suitably, the mixing may be performed for at least 30 minutes, preferably at least 60 minutes, and most preferably at least 90 minutes.

Referring to FIG. 1, the process further comprises separating at least a portion of non-aqueous phase 110 from first combined solution 106. While FIG. 1 shows aqueous phase 108 also being separated, this is optional and is included in FIG. 1 to illustrate the two phases (108 and 110) being described. While the steps of forming first combined solution 106 and separating non-aqueous phase 110 can be performed separately, they can suitably be carried out at least partially (and/or fully) together via a liquid-liquid extraction or separation process as further described below.

After at least a portion of the xylose in first combined solution 106 has been allowed to react to form xylose-diboronate esters and first combined solution 106 comprises aqueous phase 108 and non-aqueous phase 110, the phases 108 and 110 can be separated using suitable methods, such as liquid-liquid extraction or separation methods, suitably in co-current flow and preferably counter-current flow. It is understood by one of ordinary skill that aqueous phase 108 can contain an amount of water-insoluble boronic acid and water-insoluble solvent from extraction solution 104 but the concentration of water in aqueous phase 108 is higher than the concentration of water-insoluble components from extraction solution 104. Similarly, it is understood that non-aqueous phase 110 can contain an amount of water but the concentration of water-insoluble boronic acid and water-insoluble solvent in non-aqueous phase 110 is higher than the concentration of water.

Such extraction or separation methods can be performed using a series of mixers-decanters but can also be performed in a unit or series of units that integrates mixing and decanting and is preferably operated in counter current flow. Such unit can optionally contain internal components to facilitate the mixing and decanting, including stationary components (trays, random or structured packings) or agitators (e.g., rotating or oscillating disks).

Referring to FIG. 1, forming of first combined solution 106 and separating of non-aqueous phase 110 can be performed at ambient temperature (at least 20 °C), and optionally either or both can be performed at elevated temperature, such as at least 30 °C, at least 50 °C, or at least 90 °C. One exemplary factor to consider in selecting an elevated temperature is that higher temperatures will allow shorter extraction times to facilitate the rate of reaction of xylose with boronic acid, and correspondingly, the conversion from xylose to xylose-diboronate ester in first combined solution 106. There are other factors for consideration known to one of ordinary skill in selecting conditions to perform step 106 and/or step 108, such as energy requirements.

In one embodiment, forming of first combined solution 106 and separating of non-aqueous phase 110 can be performed isothermally (e.g., temperature is the same for both steps). Additionally, or alternatively, they can be performed under a temperature gradient. One suitable way of providing a temperature gradient is to provide xylose-containing solution 102 at a higher temperature than extraction solution 104. For instance, the temperature of xylose-containing solution 102 can be at least 5 °C higher than the temperature of extraction solution 104, preferably at least 10 °C higher, more preferably at least 15 °C higher, and most preferably at least 20 °C higher. If this option is selected, xylose-containing solution 102 and extraction solution 104 are preferably combined in counter-current flow so that extraction solution 104 is warmed up and the xylose-containing solution is cooled down while contacting one another to allow the xylose to be extracted from the warmer xylose-containing solution 104 to the cooler extraction solution 104. In this option, the combination of the counter-current flow and the temperature gradient combines (1) a relatively higher xylose extraction rate from the hot and xylose-rich aqueous phase 108 and (2) a lower amount of the water-insoluble solvent and boronic acid (BA) in the cold and xylose-lean aqueous phase 108, both (1) and (2) as compared to forming of first combined solution 106 without counter-currently flow and/or temperature gradient. Non-aqueous phase 110 is further processed to recovery the furfural while the separated aqueous phase 108 is not. As such, an option to lower the amount of water-insoluble solvent and BA in aqueous phase 108 results in a higher amount of non-aqueous solvent and BA remaining in non-aqueous phase 110. That is, use of a temperature gradient and counter-current flows allow for a lower loss of water-insoluble solvent and BA in the systems and processes described herein.

Referring to FIG. 1, the at least a portion of non-aqueous phase 110, preferably at least 20% of non-aqueous phase 110, is separated from first combined solution 106 using one or more suitable separation methods as described above. More preferably, at least 50%, including at least 70%, or at least 90% of non-aqueous phase 110 is separated from first combined solution 106. Preferably, such separation is achieved using at least liquid-liquid separation via gravity whereby phases 108 and 110 are retained in a separator at least an amount of time that allows phases 108 and 110 to separate by the differences in density of phases 108 and 110, with aqueous phase 108 typically being below (it generally has a higher density than) non-aqueous phase 110. It is known to one of ordinary skill that such separation via liquid-liquid separation can also be facilitated by enhanced gravity using, for example, hydrocyclone and centrifugation devices. It is understood that one of ordinary skill would be capable of selecting the suitable separator specifications (such as size, configuration, including whether and which internal components to include to facilitate mixing and decanting, arrangement, including whether to use dedicated units (e.g., mixer-decanter) and/or integration with the extraction unit, and whether and which enhanced gravity devices to include) and suitable amount of time to achieve an equilibrium condition between phases 108 and 110 at the temperature and pressure of separation of first combined solution 106 to allow for separation and/or removal of at least a portion of non-aqueous phase 110 from first combined solution 106, which effectively removes non-aqueous phase 110 from aqueous phase 108. As noted above, the xylose-diboronate ester has lower solubility in water, which means non-aqueous phase 110 comprises a greater portion of the xylose-diboronate ester than aqueous phase 108. Separation of non-aqueous phase 110 also separates or removes a greater portion of the xylose-diboronate ester from first combined solution 106.

**Ionic Conversion solution**

When the xylose-diboronate esters come into contact and react with water molecules generally from the ionic conversion solution as described herein, the xylose-diboronate esters are converted to furfural via hydrolysis to xylose and subsequent dehydration to furfural.

Accordingly, referring to FIG. 1, the process further comprises providing ionic conversion solution 142 which comprises a water-soluble solvent and water and has a pH of less than or equal to 4, preferably less than or equal to 3, more preferably less than or equal to 2, and most preferably less than or equal to 1.

In particular, conversion solution 142 has a pH of less than or equal to 4 and comprises one or more salts, each salt comprises a plurality of ions selected from the group consisting of an anion, a cation, and a combination thereof. Conversion solution 142 has a calculated molar ionic strength of at least 1, preferably at least 2, more preferably at least 3, and most preferably at least 4. The molar ionic strength is determined according to equation (1):

$$I = ½ \, sum(c_i * z_i^2) \qquad\qquad (1)$$

wherein

I is the calculated molar ionic strength;
i is an ion of the salt(s) in the conversion solution
$c_i$ is the molar concentration of ion i (M, mol/L) in conversion solution 142, and
$z_i$ is the charge number of ion i

and the summation is over ions from the one or more salts. As known by one of ordinary skill, ions are selected from the group consisting of cations, anions, and a combination thereof.

Suitably, any acid may be used to provide the ionic conversion solution with a pH of less than 4. One of ordinary skills would be able to determine the type and corresponding amount of an acid to achieve the desired pH of ionic conversion solution 142. For instance, one or more inorganic acids, one or more organic acids, or any combination thereof can be used to provide an ionic conversion solution with pH of less than 4. Examples of suitable oxygenated acids include, but are not limited to, phosphoric acid, pyrophosphoric acid, phosphorus acid, nitric acid, sulfuric acid, sulphurous acid, hypochloric acid and chlorate acid. Examples of non-oxygenated acids include, but are not limited to, hydrochloric acid and hydrobromic acid. Suitable organic acids include methane sulfonic acid, formic acid, acetic acid, mono/di/trifluoro acetic acid, mono/di/trichloro acetic acid, and any combinations thereof. Preferably, inorganic acids such as HCl or $H_2SO_4$ are used.

As known to one of ordinary skill in the art, the amount of acid to provide ionic conversion solution 142 with a pH of less than 4 varies depending on the strength of the acid(s) used. A greater amount of a weaker acid would be needed to achieve the same pH as compared to a stronger acid. Suitably, a pH of less than 4 corresponds to an acid concentration of at least 0.1 mMol/L in ionic conversion solution 142 for a strong acid such as $H_2SO_4$ or HCl. If weaker acid(s) are selected, then ionic conversion solution 142 would have a higher concentration of the weaker acid(s) to achieve the corresponding pH of less than 4.

While the acid(s) in ionic conversion solution 142 contributes ions, equation (1) for determining the calculated molar ionic strength I does not include ions coming from such acid(s) because most of the ionic strength typically comes from the salt(s) when present. As such, reference is made to the "calculated" molar ionic strength. Nevertheless, optionally, one of ordinary skill can achieve a conversion solution with an ionic strength of at least 1 using acid(s) without adding a salt to ionic conversion solution 142 or adding a minimal amount of salt. The molar ionic strength can be determined modifying equation (1) to account for the ions from the acid(s) by taking the summation of ions from the acids and any minimal amount of salt, as applicable.

As described, ionic conversion solution 142 comprises one or more salts. The salt can be organic or inorganic and is soluble in water and insoluble in first non-aqueous phase 110 at a temperature in a range from 20 °C and up to 200 °C (or temperature $T_r$ further referenced below). As used herein, "soluble" and its grammatical variations have their ordinary respective meanings, which describe the amount of a substance that will dissolve in a given amount of solvent at a specified temperature. A salt that is soluble in water is one that has with an octanol-water partition coefficient LogP of at most 0, preferably at most (-0.5), more preferably at most (-1.0). As used herein, "insoluble" and its grammatical variations have their ordinary respective meanings, which describe the relatively low amount of a substance that will dissolve in a given amount of solvent at a specified temperature as compared to a "soluble" substance or one with higher solubility in that solvent under similar conditions. A salt that is insoluble in first non-aqueous phase 110 at a temperature in a range from 20 degrees C and up to the reaction temperature is one that has preferably up to 5 wt.% solubility, including up to 2 wt.% solubility, up to 1 wt.% solubility, up to 0.5 wt.% solubility, or up to 0.1 wt.% solubility at such temperature range.

The salt(s) comprise ions selected from the group consisting of monovalent cations, monovalent anions, divalent cations, divalent anions, trivalent cations, trivalent anions, and any combination thereof. Preferably the salt(s) contains the same anion as the acid in conversion solution 142, such as $Na_2SO_4$, $NaHSO_4$ or $MgSO_4$ with $H_2SO_4$ as acid or NaCl or MgCh with HCl as acid as an option to mitigate neutralizing effects by a salt that contains an anion that is different than the anion of the acid, as known by one of ordinary skill.


**Second combined solution: dehydration reaction**

Referring to FIG. 1, the process further comprises combining an amount of conversion solution 142 with an amount of non-aqueous phase 110 to form second combined solution 144. The ratio of conversion solution 142 to non-aqueous phase 110 in second combined solution 144 is in a range from 0.1 to 10.0, preferably from 0.3 to 5.0, and more preferably from 0.5 to 2.0, by weight, respectively.

Preferably, at least 20% of the separated non-aqueous phase 110 is combined with conversion solution 142, more preferably at least 50%, including at least 70%, at least 90%, or all of the separated non-aqueous phase 110 is combined with conversion solution 142. The ratio of ionic conversion solution 142 to non-aqueous phase 110 in second combined solution 144 is in a range from 0.1 to 10.0, preferably from 0.3 to 3.0, and more preferably from 0.5 to 1.0, by volume, respectively. Notably, second combined solution 144 comprises: furfural, water-insoluble boronic acid and water-insoluble solvent from extraction solution 104, and ions from the acid and/or salt in ionic conversion solution 142.

Amongst other things, the inventors have found that combining non-aqueous phase 110 (which comprises the xylose-diboronate ester) with an ionic conversion solution with a pH of less than or equal to 4 and a molar ionic strength of at least 1 can provide for improved conversion of the xylose-diboronate ester, improved furfural selectivity, and improved subsequent product processing (described further below and elsewhere) than a conversion solution not as described herein.

As used herein, "conversion" has its ordinary meaning, which includes meaning the fraction of starting molecules that have reacted and been converted to something else. Also, "yields" has its ordinary meaning, which includes meaning the fraction of feed molecules that become the desired product rather than by-products at the end of the process. As used herein, "selectivity" has its ordinary meaning, which includes the fraction of converted molecules that become the desired product rather than by-products at the end of the process. For example, and as further demonstrated in Examples 2 and 3 below, if a reaction begins with 1 mole of $BA_2X$ and an analysis of the reaction product mixture shows 0.5 mol of unconverted $BA_2X$, then the $BA_2X$ conversion is 50% because 50% of the starting $BA_2X$ has been converted to something else. If the analysis of the reaction product mixture shows that 90% of the reacted $BA_2X$ become furfural while the remaining 10% become by-products, then the furfural yields would be 45% (0.5*0.9) while the furfural selectivity is 90%.

Without being bound by theory, it is believed that in second combined solution 144, the ions from the salt(s) in ionic conversion solution 142 interact with water molecules through solvation forces, thereby reducing the overall availability of water molecules in second combined solution 144, which reduces the solubility of furfural, BA, $BA_2X$ and the

water-insoluble solvent in the aqueous phase in second combined solution 144 (eventually part of aqueous phase 146). The reduced solubility of furfural in turn decreases undesired degradation reactions of furfural with itself or with unconverted sugars in second combined solution 144, which leads to higher selectivity.

Because furfural, BA and $BA_2X$ are insoluble in the non-aqueous phase of second combined solution 144 (eventually part of non-aqueous phase 148), the additional benefit of reduced solubility in the aqueous phase means less of these compounds remain dissolved in the aqueous phase and more go into the non-aqueous phase, thereby facilitating subsequent product processing, which includes (i) recovery of the furfural and (ii) recycling of certain portion(s) of the water-insoluble solvent (with dissolved BA) and the ionic conversion solution as further described herein, both with lower losses of the respective constituents.

Referring to FIG. 1, the process further comprises heating the second combined solution to a temperature, $T_r$, of at least 130 °C, preferably at least 150 °C, more preferably at least 170 °C, and most preferably at least 200 °C, wherein such heating converts at least a portion of the xylose-diboronate ester into furfural. The reaction is carried out under pressure, where the pressure is at least autogenous, which has its ordinary meaning.

Referring to FIG. 1 and as noted above, when the xylose-diboronate esters from non-aqueous phase 110 in second combined solution 144 come into contact and react with water molecules generally from conversion solution 142, the xylose-diboronate esters are converted to furfural via a dehydration reaction.

By heating second combined solution 144 to temperature $T_r$, at least 20 mol% of the xylose-diboronate ester in second combined solution 144 is converted into furfural, more preferably at least 50 mol%, including at least 80 mol% of the xylose-diboronate ester in second combined solution 144 is converted into furfural, preferably at least 90 mol%.

Alternatively, non-aqueous phase 110 and aqueous phase 142 can be heated up to the reaction temperature $T_r$ individually and subsequently contacted or combined to form second combined solution 144 to allow the reaction to proceed. That is, one of ordinary skill understands that the reaction involves contact between non-aqueous phase 110 and ionic conversion solution 142 at temperature $T_r$. The order in which this is achieved (whether by forming second combined solution 144 which is then heated to temperature $T_r$ or heating the components of second combined solution 144 to separately prior to forming second combined solution 144 with a temperature $T_r$) is a design choice.

Suitably, second combined solution 144 may be heated at temperature $T_r$ for at least 1 minute, preferably at least 10 minutes, more preferably at least 30 minutes, most preferably at least 60 minutes, and preferably up to 10 hours, more preferably up to 5 hours, and most preferably up to 3 hours. For instance, the amount of time second combined solution 144 is preferably heated at temperature $T_r$ is in a range from 10 minutes to 10 hours, more preferably from 30 minutes to 5 hours, and most preferably from 60 minutes to 3 hours.

Referring to FIG. 1, second combined solution 144 is preferably mixed at least a portion of the time it is heated at temperature $T_r$, said mixing is performed using suitable methods such as at least those described herein. Such mixing can further facilitate contact between water molecules and the xylose-diboronate esters for conversion to furfural. Suitable mixing methods include mixing, stirring, static mixer, turbulent flow, jet loop, etc. Suitably, the mixing may be performed for at least 30 minutes, preferably at least 60 minutes, and most preferably at least 90 minutes.

After an amount of time, the heated second solution 144 comprises a second aqueous phase (illustrated as aqueous phase 146) comprising water and at least 90% of the ions in the second combined solution. Heated second solution 144 further comprises a second non-aqueous phase (illustrated as non-aqueous phase 148) comprising greater than 50% (preferably at least 75%, and more preferably at least 95%) of the water-insoluble solvent, at least 50% (preferably at least 75%) of the water-insoluble boronic acid, and at least 50% of the furfural in the second combined solution.

**Subsequent product processing**

It is understood by one of ordinary skill that aqueous 146 can contain an amount of water-insoluble boronic acid and water-insoluble solvent, but the concentrations of water and water-soluble solvent are lower than those in non-aqueous phase 110. Similarly, it is understood that non-aqueous phase 148 can contain an amount of water and water-soluble solvent but the concentrations of water-insoluble solvent and water-insoluble boronic acid are higher than those in conversion solution 142.

Non-aqueous phase 148 comprises at least a portion of the produced furfural in second combined solution 144, preferably at least 50%, more preferably at least 70%, and most preferably at least 85% of the produced furfural in second combined solution 144. At least a portion of the furfural in non-aqueous phase 148 can be recovered by suitable methods, such as distillation where the furfural is part of an overhead product.

Suitable methods to recover the furfural in non-aqueous phase 148 includes separating at least a portion of non-aqueous phase 148 from second combined solution 144 for subsequent recovery processes. For instance, referring to FIG. 1, at least non-aqueous phase 148 and aqueous phase 146 may be separated from cooled second combined solution 144. At least a portion (preferably greater than 50%) of non-aqueous phase 148 may be provided to a distillation unit to recover at least a portion of the furfural as overhead product 120 while leaving a majority (greater than 50%) of non-aqueous phase 148 comprising boronic acid and water-insoluble solvent as bottom product 122. Suitably, additionally or alternatively, permeation or affinity separation (not shown) can be used to recover at least a portion of the produced furfural.

Referring to FIG. 1, bottom product 122 comprises at least a portion of water-insoluble boronic acid and water-insoluble solvent from extraction solution 104. Optionally, at least a portion of bottom product 122 can be recycled (i.e., reused) as part of extraction solution 104. That is, optionally, extraction solution 104 can comprise at least a portion of bottom product 122, including at least 20 wt.%, preferably at least 50 wt.%, more preferably at least 70 wt.%, and most preferably at least 90 wt.% or at least 95 wt.%.

After the dehydration reaction, the composition of aqueous phase 146 comprises (i) water in a range from 50 to 95 wt.%, (ii) more than 5 wt.% salt (which contains more than 95%, preferably more than 99%, of the salt in the second combined solution 144) and (iii) less than 8 wt.% furfural (which contains about 5% and up to 50% of the furfural produced in second combined solution 144). The composition of non-aqueous phase 148 comprises (i) non-aqueous solvent in a range from 50 wt.% and up to 95 wt.% (which contains more than 95% of the non-aqueous solvent in the second combined solution 144), (ii) BA in a range from 2 wt.% and up to 20 wt.% (which contains more than 50% of the BA in second combined solution 144), (iii) furfural in a range from 1 wt.% and up to 10 wt.% (which contains from 50% and up to 95% of the furfural produced in second combined solution 144), and (iv) less than 1 wt.% of water. Referring to FIG. 1, at least a portion (preferably greater than 50%) of aqueous phase 146 can optionally be recycled for use as part of ionic conversion solution 142.

As described, referring to FIG. 1, aspects of process allow for recycling of at least a portion (preferably greater than 50%) of (i) bottom product 122 for use as part of extraction solution 104 and/or (ii) aqueous phase 146 for use as part of ionic conversion solution 142. Such recycling reduces the consumption of costly chemicals, particularly water-insoluble solvent and water-insoluble boronic acid of extraction solution 104 and/or water-soluble solvent and inorganic acid of conversion solution 142.

Optionally, aqueous phase 108 can comprise water-insoluble solvent and water-insoluble boronic acid. Optionally, aqueous phase 108 may be further processed (not shown) to recover at least a portion of the water-insoluble solvent, or the water-insoluble boronic acid, or a combination thereof. Suitable further processing of aqueous phase 108 may include adsorption.

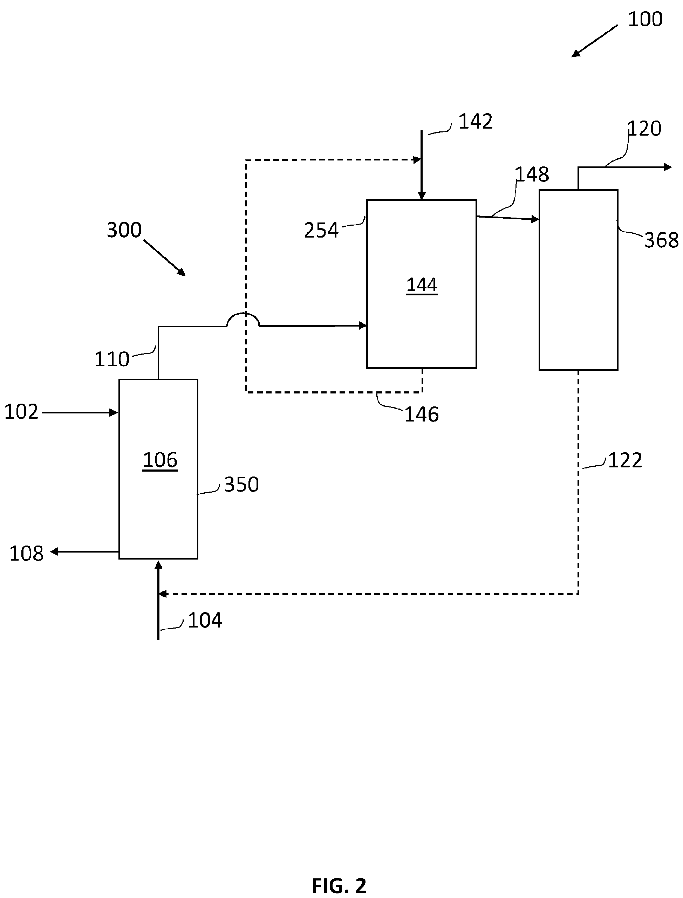

FIG. 2 illustrates one exemplary suitable embodiment of system 200 for producing furfural from xylose in accordance with this disclosure, such as process 100. As shown in FIG. 2, xylose-containing solution 102 and extraction solution 104 are provided to and combined in extraction unit 350 to form first combined solution 106. Extraction unit 350 is designed to perform liquid-liquid extraction, preferably in counter-current, and perform separation to separate non-aqueous phase 110 as described above. As shown in FIG. 2, system 200 allows for the steps of forming first combined solution 106 and separating non-aqueous phase 110 to be carried out at least partially (and/or fully) together via extraction unit 350. Extraction unit can be performed in co-current flow and preferably counter-current flow.

Extraction unit 350 can be operated isothermally for a desired amount of time at a constant temperature. Additionally, or alternatively, extraction unit 350 can be operated with a temperature gradient for a desired amount of time by providing xylose-containing solution 102 at a temperature that is at least 5 °C, at least 10 °C, at least 15 °C, or at least 20 °C, higher than the temperature of extraction solution 104. If extraction unit 350 is operated in counter-current mode with a temperature gradient, such operation can combine a higher extraction rate associated with the warmer section that is in close proximity with the inlet of extraction unit 350 for xylose-containing solution 102 (which translates to higher concentration of xylose in the portion of first combined solution 106 at that location) with low loss of extraction solution 104 in the water-rich effluent in the cooler section in close proximity with an outlet for aqueous phase 108.

Referring to FIG. 2, aqueous phase 108 and non-aqueous phase 110 are separated via extraction unit 350. Non-aqueous phase 110 comprises at least 20 mol% (and preferably more than 50 mol%) of the xylose in the xylose containing solution 102 initially provided to form first combined solution 106. The processes described herein, such as process 100 in FIG. 1 which can be performed with system 200 in FIG. 2, can be designed to have at least 90% selectivity for xylose, which means in such an embodiment, non-aqueous phase 110 comprises at least 90% and aqueous phase 108 comprises less than 10% of the xylose originally in xylose-containing solution 102.

Referring to FIG. 2, non-aqueous phase 110 is provided to dehydration unit 352. Conversion solution 142 is also provided to dehydration unit 352, and non-aqueous phase 110 and conversion solution 142 are combined in unit 352 to form second combined solution 144, respectively. Referring to FIG. 2, second combined solution 144 is heated while preferably being mixed at least a portion (or substantially all) of the time being heated in dehydration unit 352 by a suitable method and corresponding component(s) as described above. In one embodiment, second combined solution 144 is heated to a temperature of at least 130 °C in dehydration unit 352 to convert xylose-diboronate ester to furfural.

Referring to FIG. 2, second combined solution 144 is heated in dehydration unit 254 to a temperature below temperature $T_r$, where heated second combined solution 144 comprises an aqueous phase comprising water, ions, and furfural, and a non-aqueous phase comprising water-insoluble boronic acid, water-insoluble solvent, and furfural. At least a portion of such aqueous and non-aqueous phases of second combined solution 144 can be separated as stream 146 and 148, respectively, using suitable methods such as those described herein, including liquid-liquid separation. In system 200, dehydration unit 252 is also capable of performing liquid-liquid separation (or extraction) to separate aqueous phase 146 and non-aqueous phase 148 from second combined solution 144.

Referring to FIG. 2, non-aqueous phase 148 is provided to distillation unit 368 for recovery of furfural in overhead product stream 120. As shown, at least a portion (at least 50% or substantially all) of bottom product stream 122 can be recycled (or reused) as part of extraction solvent 104. At least a portion of aqueous phase 146 is recycled as part of ionic conversion solution 142.

Preferably, embodiments of the processes and systems as described herein are carried out or operated continuously for an amount of time, such as at least 6 hours.

## EXAMPLES

Embodiments of the processes and systems described herein can be further illustrated by the following exemplary, non-limiting examples

### Example 1 - xylose extraction

In Example 1, nine experiments were conducted with different water-insoluble solvent and pH as shown in Table 3 below. General experimental conditions for these nine experiments include:

- xylose-containing solution: 150 mL solution containing D-xylose (350 mM) in water that was acidified to various pH with $H_2SO_4$ and
- extraction solution: 150 mL solution containing phenyl boronic acid (PBA) in a water-insoluble solvent; the concentration of PBA and type of water-insoluble solvent are provided in Table 3 below
- the xylose-containing solutions and extraction solutions of Example 1 were each mixed in a 500 mL Erlenmeyer flask, provided with a cap, and stirred at room temperature for 2 hours, which results in an aqueous phase and a non-aqueous phase in the flasks. Subsequently, the two phases of each experiment were separated and the resulting concentrations of xylose (X) and phenylboronate xylose diester ($PBA_2X$) were quantified in each phase using $^1$H-NMR analysis in the presence of internal standard as described below. The quality of extraction is then reported as molar yield of $PBA_2X$ found in the organic phase in Table 3 below.

The aqueous phases and non-aqueous phases from Example 1 were analysed by means of $^1$H-NMR using a 400 MHz Bruker spectrometer. The aqueous phases were measured in a 1:1 $H_2O/D_2O$ mixture (in which $D_2O$ is deuterated water also called heavy water or heavy water used for the analysis) with sodium 2,2,3,3-$d_4$-trimethylsilylpropanoate (TMSP) as the internal standard and the non-aqueous phases were measured in a 1:1 mixture of toluene and toluene-$d_8$ with dioxane as the internal standard. These mixtures of aqueous and non-aqueous phases being analysed are composed by equal volumes (250 μL) of the particular aqueous or non-aqueous phase and the deuterated solvent, containing the standard.

### TABLE 3

| Extraction efficiency at various conditions (Xylose concentration in water 350 mM, room temperature, water-solvent volume ratio of 1:1.) | | | | |
|---|---|---|---|---|
| | Water-insoluble solvent | PBA (mM) in water-insoluble solvent | pH of aqueous phase | Extracted Xylose (%mol) |
| 1 | toluene | 700 | 1 | 84 |
| 2 | toluene | 350 | 1 | 47 |
| 3 | toluene | 1050 | 1 | 95 |
| 4 | toluene | 700 | 3 | 83 |
| 5 | toluene | 700 | 6 | 84 |
| 6 | toluene | 700 | 9 | 83 |
| 7 | 2-methyl naphthalene | 700 | 1 | 89 |
| 8 | n-heptane | 700 | 1 | 55 |

(continued)

| Extraction efficiency at various conditions (Xylose concentration in water 350 mM, room temperature, water-solvent volume ratio of 1:1.) | | | | |
|---|---|---|---|---|
| | Water-insoluble solvent | PBA (mM) in water-insoluble solvent | pH of aqueous phase | Extracted Xylose (%mol) |
| 9 | 1-octanol | 700 | 1 | 30 |

The experiments of Example 1 show recovery of xylose using toluene, 2-methylnaphthalene, n-heptane, and octanol as water-insoluble solution, with PBA as the water-insoluble boronic acid.

In experiments 1 and 4-9, the molar ratio of boronic acid to xylose was 2:1. Experiments 2 and 3 demonstrate a potential correlation between extraction efficiency and ratio of boronic acid to xylose, particularly that a higher molar ratio of boronic acid can produce a higher extraction rate (experiment 3) while a lower molar ratio of boronic acid also results in a lower extraction of xylose (experiment 2).

In addition, these experiments show that embodiments of the processes and systems described herein allow for xylose extraction from acidic and basic xylose-containing solutions. The experiments also demonstrate that embodiments of the processes and systems described herein allow for xylose extraction using aromatic solvents and aliphatic solvents, although the aromatic solvents (toluene and 2-methylnaphthalene) provide a better rate of extraction than the aliphatic solvents (n-heptane and octanol).

The non-aqueous phase of experiments 1 - 9 of Example 1 can be combined with a conversion solution to produce furfural (dehydration reaction) as described herein (such as conversion solution 142). Example 2 below shows experiments of such dehydration reaction.

## Example 2 - Xylose dehydration

For this analysis, the reaction of dehydration of $PBA_2X$ (a particular type of $BA_2X$ as described above) was studied by using conversion solutions that was acidified to pH=1 by addition of $H_2SO_4$. The salt in the ionic conversion solutions was $Na_2SO_4$ at various concentrations, namely, 0 M of $Na_2SO_4$, 0.5 M, 1 M, 1.3 M, and 2 M to vary the calculated ionic strength between 0 and 6, which is shown in Table 4 below. For illustration, a solution of 0.5 M of $Na_2SO_4$ will have a calculated ionic strength I of I = ½ * (2*0.5*(+1)$^2$ + 0.5*(-2)$^2$) = 1.5. Table 4 below also shows ionic strength I* which is greater than the calculated ionic strength because I* takes into consideration the ions from the acid in the respective conversion solution using the equation provided herein.

The conversion solutions with varying calculated ionic strengths were added to a representative non-aqueous phase to form second combined solution to carry out the dehydration reaction. The representative non-aqueous phase contained a $PBA_2X$ concentration of 320 mM in toluene. The molar ratio of non-aqueous phase to ionic conversion solution was 1:1. The reaction in second combined solution was run for 2 h at 200 °C and autogenous pressure.

The resulting mixture was analysed for furfural yield, furfural selectivity, and PBA2X conversion as defined below.

$$PBA_2X \text{ conversion} = \text{mole of converted } PBA_2X/\text{mole of } PBA_2X$$

$$\text{Furfural yield} = \text{mole of Furfural/mole of } PBA_2X$$

$$\text{Furfural selectivity} = \text{mole of Furfural/mole of converted } PBA_2X = \text{yield/conversion}$$

**TABLE 4**

| Effect of ionic strength on yield, selectivity, and conversion | | | | | |
|---|---|---|---|---|---|
| Experiment No. | Ionic Strength I* (M) | Calculated Ionic Strength I (M) | $PBA_2X$ Conversion (mol%) | Furfural Yield (mol%) | Furfural selectivity (mol%) |
| 1 | 0.1 | 0 | 82 | 57 | 69 |

(continued)

| Effect of ionic strength on yield, selectivity, and conversion | | | | | |
|---|---|---|---|---|---|
| Experiment No. | Ionic Strength I* (M) | Calculated Ionic Strength I (M) | PBA$_2$X Conversion (mol%) | Furfural Yield (mol%) | Furfural selectivity (mol%) |
| 2 | 1.6 | 1.5 | 74 | 58 | 78 |
| 3 | 3.1 | 3 | 69 | 60 | 86 |
| 4 | 4.1 | 4 | 65 | 60 | 93 |
| 5 | 6.1 | 6 | 58 | 51 | 88 |

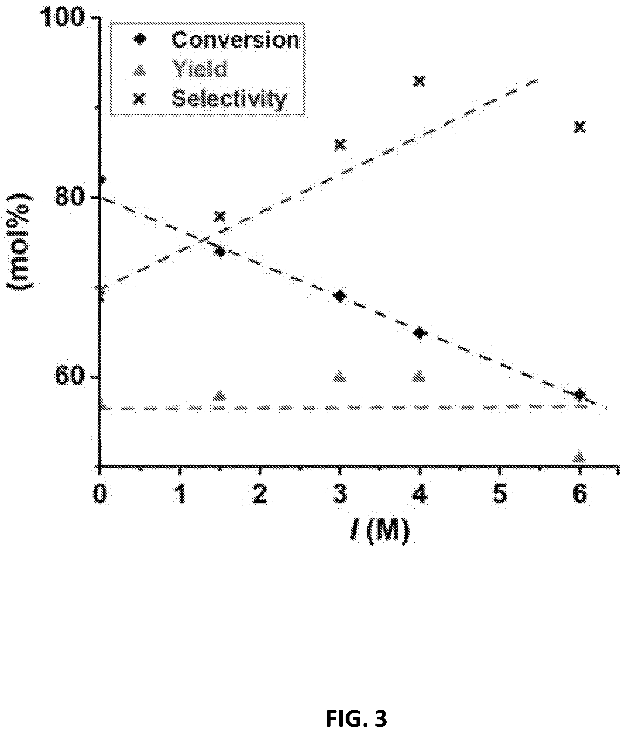

FIG. 3 is a graph of Table 4, with the calculated ionic strength, I (M), on the x-axis, and PBA$_2$X conversion, furfural yield, and furfural selectivity on the y-axis. FIG. 3 shows that PBA$_2$X conversion proceeds more slowly as the ionic strength of the conversion solution increases (from approx. 80 mol% at 0 M of sodium sulphate to approx. 60 mol% at 2 M of sodium sulphate). This observed relationship between PBA$_2$X conversion and ionic strength suggests that more reaction time can be provided to address the slower rate of PBA$_2$X conversion. While an increased ionic strength in the conversion solution can result in a slower rate of PBA$_2$X conversion, FIG. 3 also shows an increase in furfural selectivity as the ionic strength increases, whereby the selectivity was raised from approx. 70 mol% (0 M of sodium sulphate) to approx. 88-90 mol% (at 2 M of sodium sulphate). Furfural yield stayed generally unchanged, approx. 60 mol% regardless of salt concentration.

**Example 3 - Xylose dehydration**

In Example 3, conversion solutions comprising different salts and/or acids combinations and having varying ionic strengths were examined:

- Salt = MgSO$_4$; acid = H$_2$SO$_4$; pH = 1
- Salt = NaCl; acid = HCl; pH = 1
- Salt = MgCl$_2$; acid = HCl; pH = 1

The conversion solutions with varying calculated ionic strengths (as shown in Table 5) were added to a representative non-aqueous phase to form second combined solution to carry out the dehydration reaction. The representative non-aqueous phase contained a PBA$_2$X concentration of 320 mM in toluene. The molar ratio of non-aqueous phase to ionic conversion solution was 1:1. The reaction in second combined solution was run for 2 h at 200 °C and autogenous pressure.

The resulting mixture was analysed for furfural yield, furfural selectivity, and PBA$_2$X conversion as defined in Example 2.

**TABLE 5**

| Effect of ionic strength on yield, selectivity, and conversion | | | | | |
|---|---|---|---|---|---|
| Experiment No. | Ionic Strength (M) | Calculated Ionic Strength (M) | Added salt and acid | PBA Conversion (mol%) | Furfural selectivity (mol%) |
| 1 | 0.1 | 0 | H$_2$SO$_4$/n.a. | 74 | 64 |
| 2 | 0.1 | 0 | HCl/n.a. | 82 | 69 |
| 3 | 1.6 | 1.5 | H$_2$SO$_4$/Na$_2$SO$_4$ | 74 | 78 |
| 3 | 3.1 | 3 | H$_2$SO$_4$/Na$_2$SO$_4$ | 69 | 86 |
| 4 | 4.1 | 4 | H$_2$SO$_4$/Na$_2$SO4 | 57 | 90 |

(continued)

| Effect of ionic strength on yield, selectivity, and conversion | | | | | |
|---|---|---|---|---|---|
| Experiment No. | Ionic Strength (M) | Calculated Ionic Strength (M) | Added salt and acid | PBA Conversion (mol%) | Furfural selectivity (mol%) |
| 5 | 4.1 | 4 | $H_2SO_4$/$Na_2SO_4$ | 54 | 89 |
| 6 | 6.1 | 6 | $H_2SO_4$/$Na_2SO_4$ | 57 | 88 |
| 7 | 1.1 | 1 | HCl/NaCl | 81 | 71 |
| 8 | 3.1 | 3 | HCl/NaCl | 65 | 83 |
| 9 | 4.1 | 4 | HCl/NaCl | 55 | 87 |
| 10 | 6.1 | 6 | HCl/NaCl | 64 | 86 |
| 11 | 3.1 | 3 | $H_2SO_4$/$MgSO_4$ | 81 | 87 |
| 12 | 4.1 | 4 | $H_2SO_4$/$MgSO_4$ | 64 | 93 |
| 13 | 6.1 | 6 | $H_2SO_4$/$MgSO_4$ | 60 | 89 |
| 14 | 3.1 | 3 | $HCl/MgCl_2$ | 76 | 82 |
| 15 | 4.6 | 4.5 | $HCl/MgCl_2$ | 61 | 84 |
| 16 | 6.1 | 6 | $HCl/MgCl_2$ | 70 | 85 |

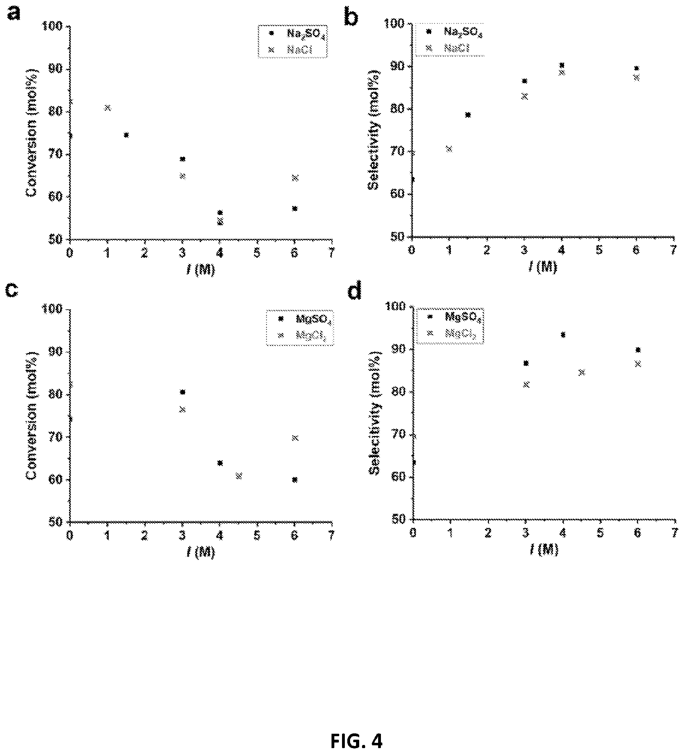

FIG. 4 is a graph of Table 5, with the calculated ionic strength, I (M), on the x-axis, and (i) $PBA_2X$ conversion in FIG. 4A and furfural selectivity in FIG. 4B on the y-axis, respectively, for the conversion solutions that contain $Na_2SO_4$ and NaCl and experiments nos. 1 and 2 for comparison at 0M, and (ii) $PBA_2X$ conversion in FIG. 4C and furfural selectivity in FIG. 4D on the y-axis, respectively, for the conversion solutions that contain $MgSO_4$ and MgCh and experiments nos. 1 and 2 for comparison at 0M.

These results show the consistency of the positive effects of ionic strength on conversion and selectivity across the different types of salt and acid.

**Example 4 - recycling of certain bottom products**

To investigate the possible losses of the different components in the biphasic system, the partitioning of salt, acid, PBA, furfural, water, and Methylnaphthalene (MN), which represents the water-insoluble solvent in the biphasic system, was studied. The biphasic solution of this Example is representative of the composition of the second combined solution 144 after the dehydration reaction that forms the furfural. The biphasic solution was prepared by combining an aqueous solution and a non-aqueous solution in 1:1 volume ratio. The aqueous solution has a pH of 1 by addition of $H_2SO_4$ and a calculated ionic strength of 3.0 M by addition of NazSOa. The non-aqueous phase solution contains MN, PBA, and furfural.

The biphasic solution was mixed to allow adequate contact amongst the various components, and the biphasic solution was subsequently allowed to settle into the two phases: aqueous and non-aqueous. The molar concentration of each phase (rather than the biphasic solution) was analysed and reported in Table 6 below. Table 6 also provides, in parentheses, the mol% of the components in each phase with respect to the biphasic solution. For example, the molar concentration (mole/L) of PBA in the aqueous phase is 129 mM which represents 22 mol% of the PBA in the biphasic solution (aqueous and non-aqueous phases).

As can be seen, this Example shows that the non-aqueous phase (representative of the second non-aqueous phase 148) contains most of the furfural and PBA in the biphasic solution, particularly 78 mol% of PBA and 88 mol% of furfural, as well as over 99 mol% of the water-insoluble solvent MN. This demonstrates that further processing of the second non-aqueous phase should recover the majority (over 50%) of the produced furfural while enabling recycling and reuse of the post-processing (e.g., post-furfural recovery) water-insoluble solvent as described herein. This Example demonstrates the minimal loss of the water-insoluble solvent to the aqueous phase.

While the aqueous phase contains a minor fraction of PBA (22 mol%), Furfural (12 mol%), and MN (<0.05 mol%), these desirable components have the option to remain in the system through recycling of the aqueous phase for use as part of the ionic conversion solution. Moreover, over 99 mol% of the Na and S, as applicable, from both the salt ($Na_2SO_4$) and acid ($H_2SO_4$) remains with the aqueous phase. This indicates nearly all the salt is kept within the second aqueous phase for recycling to the dehydration step and a minimal amount of salt remains in the second non-aqueous phase. This is a positive indication for the option of recycling the post-processing water-insoluble solvent is recycled as part of the extraction solution in the xylose-extraction step (first combined solution) as there should be minimal salt contaminant from the recycle stream.

**TABLE 6**

| Partitioning of the composition of the aqueous and non-aqueous phases two phases that form the biphasic solution prepared according to example 4. | | |
|---|---|---|
| | **Aqueous Phase** | **Non-aqueous Phase** |
| **PBA** | 129 mM (22 mol%) | 566 mM (78 mol%) |
| **Furfural** | 46 mM (12 mol%) | 352 mM (88 mol%) |
| **MN** | 2 mM (0.03 mol%) | 7.03 M (>99 mol%) |
| **water** | 55.0 M (>99 mol%) | 66 mM (0.12 mol%) |
| **Salt** (based on 2Na) | 1 M (>99 mol%) | 2 mM (0.2 mol%) |

**Claims**

1.  A method for producing furfural comprising:

    a. providing a xylose-containing solution comprising xylose in an amount of greater than or equal to 0.5 wt.%, wherein the xylose-containing solution is an aqueous solution;
    b. providing an extraction solution comprising a water-insoluble boronic acid (BA: $R\text{-}B(OH)_2$) and a water-insoluble solvent;
    c. combining the xylose-containing solution with the extraction solution to provide a first combined solution, wherein the ratio of boronic acid to xylose in the first combined solution is greater than 1:1 molar, respectively, and wherein the first combined solution comprises a first aqueous phase and a first non-aqueous phase, said non-aqueous phase comprising at least a portion of the xylose as xylose-diboronate ester ($BA_2X$);
    d. separating at least a portion of the first non-aqueous phase from the first combined solution;
    e. providing an ionic conversion solution having a pH of less than or equal to 4, said ionic conversion solution comprising one or more salts, wherein each salt comprises a plurality of ions selected from the group consisting of an anion, a cation, and a combination thereof, wherein the conversion solution has a calculated molar ionic strength of at least 1, preferably at least 2, more preferably at least 3, and most preferably at least 4, wherein the calculated molar ionic strength is determined according to equation (1):

$$I = \tfrac{1}{2} \, \text{sum}(c_i * z_i^2) \qquad (1)$$

    wherein

    I is the calculated molar ionic strength;
    i is an ion of the salt(s)
    $c_i$ is the molar concentration of ion i (M, mol/L) in the ionic conversion solution, and
    $z_i$ is the charge number of ion i
    and the summation is over ions from the one or more salts;

f. combining at least a portion of the first non-aqueous phase with the ionic conversion solution in a ratio of conversion solution to the non-aqueous phase in a range from 0.1 and up to 10.0 by volume, respectively, preferably from 0.3 and up to 3 by volume, and more preferably from 0.5 and up to 1, to form a second combined solution;

g. providing the second combined solution with a reaction temperature of at least 130 °C, preferably at least 150 °C, more preferably at least 170 °C, and most preferably at least 200 °C to form heated second combined solution to convert at least a portion of the xylose-diboronate ester into furfural, wherein the heated second combined solution comprises

a second aqueous phase comprising water and at least 90% of the ions in the second combined solution, and a second non-aqueous phase comprising greater than 50% of the water-insoluble solvent, greater than 50% of the water-insoluble boronic acid, and at least 50% of the furfural in the second combined solution;

h. separating at least a portion of the second non-aqueous phase from the second combined solution; and
i. recovering at least a portion of the furfural from the second non-aqueous phase.

2. The method of any preceding claim, wherein the salt is an organic salt, or an inorganic salt, or a combination thereof, wherein the salt is water soluble and solvent insoluble at temperature in a range of 20 °C to 200 ° C.

3. The method of claim 3, wherein the salt and acid combination is selected from the group consisting of (i) $Na_2SO_4$ or $MgSO_4$ with $H_2SO_4$, and(ii) NaCl or MgCh with HCl.

4. The method of any preceding claim, wherein the pH of the conversion solution is provided by using at least an acid selected from the group consisting of an organic acid, an inorganic acid, and a combination thereof.

5. The method of claim 4, wherein the acid is an inorganic acid selected from the group consisting of HCl, $H_2SO_4$, $H_3PO_4$ , and any combination thereof.

6. The method of claim 5, wherein at least a portion of the first aqueous phase in (c) comprises water-insoluble solvent, and water-insoluble boronic acid, said method further comprising:
further processing at least a portion of the separated first aqueous phase to recover at least a portion of the water-insoluble solvent, or the water-insoluble boronic acid, or a combination thereof.

7. The method of any preceding claim, wherein step (g) of providing the second combined solution with the reaction temperature comprises:

heating the first non-aqueous phase to the reaction temperature;
heating the ionic conversion solution to the reaction temperature; and
combining the heated first non-aqueous phase with the heated ionic conversion solution to form the heated second combined solution.

8. The method of any preceding claim, further comprising performing at least a portion of steps (c) and (d) in a liquid-liquid extraction unit in counter-current operation, wherein the xylose-containing solution is provided at a higher temperature than the temperature of the extraction solution.

9. The method of any preceding claim, wherein the water-insoluble boronic acid has up to 5 wt.% solubility in water at 20 °C.

10. The method of any preceding claim, wherein the water-insoluble boronic acid is selected from the group consisting of phenylboronic acid, 4-biphenylboronic acid, 4-butylphenyl boronic acid, 4-tert-Butylphenyl boronic acid, 4-ethylphenyl boronic acid, 2-naphthylboronic acid, naphthalene-1-boronic acid, o-tolylboronic acid, m-tolylboronic acid, (2-methylpropyl) boronic acid, butylboronic acid, octylboronic acid, phenethyl boronic acid, cyclohexyl boronic acid, and any combination thereof.

11. The method of any preceding claim, wherein the water-insoluble solvent has up to 5 wt.% solubility in water at 20°C.

12. The method of any preceding claim, wherein the water-insoluble solvent is selected from the group consisting of benzoic acid, cresol (m), di-isopropyl ether, terephthalic acid, diethylene glycol diethyl ether, anisole, salicylic acid, 2,6 xylenol, 4Et-phenol, toluene, benzofuran, ethylbenzene, octanoic acid, 1-methylnaphtalene, nitrobenzene, guaiacol, heptane, 1-octanol, and methyl isobutyl ketones, an any combination thereof.

13. The method of any preceding claim, wherein at least one of the water-insoluble boronic acid and water-insoluble solvent has a boiling point higher than that of furfural, preferably at least 2 °C higher.

14. The method of any one of claims 10, 12, and 13, wherein step (i) comprises providing at least a portion of the second non-aqueous phase from (h) to a distillation process to recover an overhead product comprising furfural and a bottom product comprising water-insoluble solvent and water-insoluble boronic acid.

15. The method of claim 14, further comprising providing at least a portion of the bottom product for use as part of the extraction solution.

**Patentansprüche**

1. Verfahren zum Produzieren von Furfural, umfassend:

a. Bereitstellen einer xylosehaltigen Lösung, umfassend Xylose in einer Menge von größer als oder gleich 0,5 Gew.-%, wobei die xylosehaltige Lösung eine wässrige Lösung ist;
b. Bereitstellen einer Extraktionslösung, umfassend eine wasserunlösliche Boronsäure (BA: R-B(OH)$_2$) und ein wasserunlösliches Lösungsmittel;
c. Kombinieren der xylosehaltigen Lösung mit der Extraktionslösung, um eine erste kombinierte Lösung bereitzustellen, wobei das Verhältnis von Boronsäure zu Xylose in der ersten kombinierten Lösung jeweils größer als 1 : 1 molar ist, und wobei die erste kombinierte Lösung eine erste wässrige Phase und eine erste nicht wässrige Phase umfasst, die nicht wässrige Phase umfassend mindestens einen Anteil der Xylose als Xylose-Diboronatester (BA$_2$X);
d. Abscheiden von mindestens einem Anteil der ersten nicht wässrigen Phase von der ersten kombinierten Lösung;
e. Bereitstellen einer Ionenumwandlungslösung, die einen pH-Wert von kleiner als oder gleich 4 aufweist, die Ionenumwandlungslösung umfassend ein oder mehrere Salze, wobei jedes Salz eine Vielzahl von Ionen umfasst, die aus der Gruppe ausgewählt ist, bestehend aus einem Anion, einem Kation und einer Kombination davon, wobei die Umwandlungslösung eine berechnete molare Ionenstärke von mindestens 1, vorzugsweise mindestens 2, mehr bevorzugt mindestens 3 und am meisten bevorzugt mindestens 4 aufweist, wobei die berechnete molare Ionenstärke gemäß Gleichung (1) bestimmt wird:

$$I = \tfrac{1}{2}\ \text{sum}(c_i\ *\ z_i{}^2) \qquad (1)$$

wobei

I die berechnete molare Ionenstärke ist;
i ein Ion des Salzes/der Salze ist
$c_i$ die molare Konzentration von Ion i (M, mol/L) in der Ionenumwandlungslösung ist und
$z_i$ die Ladungszahl von Ion i ist
und die Summation über Ionen aus dem einen oder den mehreren Salzen erfolgt;

f. Kombinieren von mindestens einem Anteil der ersten nicht wässrigen Phase mit der Ionenumwandlungslösung in einem Verhältnis von Umwandlungslösung zu der nicht wässrigen Phase in einem Bereich von jeweils 0,1 und bis zu 10,0 nach Volumen, vorzugsweise von 0,3 und bis zu 3 nach Volumen und mehr bevorzugt von 0,5 und bis zu 1, um eine zweite kombinierte Lösung auszubilden;
g. Bereitstellen der zweiten kombinierten Lösung bei einer Reaktionstemperatur von mindestens 130 °C, vorzugsweise mindestens 150 °C, mehr bevorzugt mindestens 170 °C und am meisten bevorzugt mindestens 200 °C, um eine erhitzte zweite kombinierte Lösung auszubilden, um mindestens einen Anteil des Xylose-Diboronatesters in Furfural umzuwandeln, wobei die erhitzte zweite kombinierte Lösung umfasst eine zweite wässrige Phase, umfassend Wasser und zu mindestens 90 % die Ionen in der zweiten kombinierten Lösung, und eine zweite nichtwässrige Phase, umfassend zu mehr als 50 % das wasserunlösliche Lösungsmittel, zu mehr als 50 % die wasserunlösliche Boronsäure und zu mindestens 50 % das Furfural in der zweiten kombinierten Lösung;

h. Abscheiden von mindestens einem Anteil der zweiten nicht wässrigen Phase von der zweiten kombinierten Lösung; und

i. Rückgewinnen von mindestens einem Anteil des Furfurals von der zweiten nicht wässrigen Phase.

2. Verfahren nach einem der vorstehenden Ansprüche, wobei das Salz ein organisches Salz oder ein anorganisches Salz oder eine Kombination davon ist, wobei das Salz bei einer Temperatur in einem Bereich von 20 °C bis 200 °C wasserlöslich und lösungsmittelunlöslich ist.

3. Verfahren nach Anspruch 3, wobei die Salz- und Säurekombination aus der Gruppe ausgewählt ist, bestehend aus (i) $Na_2SO_4$ oder $MgSO_4$ mit $H_2SO_4$ und (ii) NaCl oder $MgCl_2$ mit HCl.

4. Verfahren nach einem der vorstehenden Ansprüche, wobei der pH-Wert der Umwandlungslösung durch Verwenden von mindestens einer Säure bereitgestellt wird, die aus der Gruppe ausgewählt ist, bestehend aus einer organischen Säure, einer anorganischen Säure und einer Kombination davon.

5. Verfahren nach Anspruch 4, wobei die Säure eine anorganische Säure ist, die aus der Gruppe ausgewählt ist, bestehend aus HCl, $H_2SO_4$, $H_3PO_4$ und einer beliebigen Kombination davon.

6. Verfahren nach Anspruch 5, wobei mindestens ein Anteil der ersten wässrigen Phase in (c) wasserunlösliches Lösungsmittel und wasserunlösliche Boronsäure umfasst, das Verfahren ferner umfassend: weiteres Verarbeiten von mindestens einem Anteil der abgeschiedenen ersten wässrigen Phase, um mindestens einen Anteil des wasserunlöslichen Lösungsmittels oder der wasserunlöslichen Boronsäure oder einer Kombination davon zurückzugewinnen.

7. Verfahren nach einem der vorstehenden Ansprüche, wobei Schritt (g) des Bereitstellens der zweiten kombinierten Lösung mit der Reaktionstemperatur umfasst:

Erhitzen der ersten nicht wässrigen Phase auf die Reaktionstemperatur;
Erhitzen der Ionenumwandlungslösung auf die Reaktionstemperatur; und
Kombinieren der erhitzten ersten nicht wässrigen Phase mit der erhitzten Ionenumwandlungslösung, um die erhitzte zweite kombinierte Lösung auszubilden.

8. Verfahren nach einem der vorstehenden Ansprüche, ferner umfassend ein Durchführen von mindestens einem Anteil der Schritte (c) und (d) in einer Flüssig-Flüssig-Extraktionseinheit in einem Gegenstrombetrieb, wobei die xylosehaltige Lösung bei einer höheren Temperatur als die Temperatur der Extraktionslösung bereitgestellt wird.

9. Verfahren nach einem der vorstehenden Ansprüche, wobei die wasserunlösliche Boronsäure bei 20 °C eine Löslichkeit in Wasser von bis zu 5 Gew.-% aufweist.

10. Verfahren nach einem der vorstehenden Ansprüche, wobei die wasserunlösliche Boronsäure aus der Gruppe ausgewählt ist, bestehend aus Phenylboronsäure, 4-Biphenylboronsäure, 4-Butylphenylboronsäure, 4-tert-Butylphenylboronsäure, 4-Ethylphenylboronsäure, 2-Naphthylboronsäure, Naphthalin-1-boronsäure, o-Tolylboronsäure, m-Tolylboronsäure, (2-Methylpropyl)boronsäure, Butylboronsäure, Octylboronsäure, Phenethylboronsäure, Cyclohexylboronsäure und einer beliebigen Kombination davon.

11. Verfahren nach einem der vorstehenden Ansprüche, wobei das wasserunlösliche Lösungsmittel bei 20 °C eine Löslichkeit in Wasser von bis zu 5 Gew.-% aufweist.

12. Verfahren nach einem der vorstehenden Ansprüche, wobei das wasserunlösliche Lösungsmittel aus der Gruppe ausgewählt ist, bestehend aus Benzoesäure, Kresol (m), Diisopropylether, Terephthalsäure, Diethylenglykoldiethylether, Anisol, Salicylsäure, 2,6-Xylenol, 4Et-Phenol, Toluol, Benzofuran, Ethylbenzol, Octansäure, 1-Methylnaphthalin, Nitrobenzol, Guajacol, Heptan, 1-Octanol und Methylisobutylketonen und einer beliebigen Kombination davon.

13. Verfahren nach einem der vorstehenden Ansprüche, wobei mindestens eines der wasserunlöslichen Boronsäure und des wasserunlöslichen Lösungsmittels einen Siedepunkt aufweist, der höher als der von Furfural ist, vorzugsweise mindestens 2 °C höher.

**14.** Verfahren nach einem der Ansprüche 10, 12 und 13, wobei Schritt (i) das Bereitstellen von mindestens einem Anteil der zweiten nicht wässrigen Phase aus (h) an einen Destillationsprozess umfasst, um ein Kopfprodukt, umfassend Furfural, und ein Bodenprodukt, umfassend wasserunlösliches Lösungsmittel und wasserunlösliche Boronsäure, zurückzugewinnen.

**15.** Verfahren nach Anspruch 14, ferner umfassend das Bereitstellen von mindestens einem Anteil des Bodenprodukts zur Verwendung als Teil der Extraktionslösung.

## Revendications

**1.** Procédé de production de furfural comprenant :

a. la fourniture d'une solution contenant du xylose comprenant du xylose en une quantité supérieure ou égale à 0,5 % en poids, dans lequel la solution contenant du xylose est une solution aqueuse ;
b. la fourniture d'une solution d'extraction comprenant un acide boronique non hydrosoluble (BA : $R\text{-}B(OH)_2$) et un solvant non hydrosoluble ;
c. la combinaison de la solution contenant du xylose avec la solution d'extraction pour obtenir une première solution combinée, dans lequel le rapport entre l'acide boronique et le xylose dans la première solution combinée est supérieur à 1:1 molaire, respectivement, et dans lequel la première solution combinée comprend une première phase aqueuse et une première phase non aqueuse, ladite phase non aqueuse comprenant au moins une partie du xylose en tant que xylose-ester de diboronate ($BA_2X$) ;
d. la séparation d'au moins une partie de la première phase non aqueuse de la première solution combinée ;
e. la fourniture d'une solution de conversion ionique ayant un pH inférieur ou égal à 4, ladite solution de conversion ionique comprenant un ou plusieurs sels, dans lequel chaque sel comprend une pluralité d'ions choisis dans le groupe constitué d'un anion, d'un cation et d'une combinaison de ceux-ci, dans lequel la solution de conversion a une force ionique molaire calculée d'au moins 1, de préférence d'au moins 2, plus préférablement d'au moins 3, et le plus préférablement d'au moins 4, dans lequel la force ionique molaire calculée est déterminée selon l'équation (1) :

$$I = \tfrac{1}{2}\ \texttt{somme}(c_i\ *\ Z_i^2) \qquad (1)$$

dans laquelle

I est la force ionique molaire calculée ;
i est un ion du (des) sel(s)
ci est la concentration molaire de l'ion i (M, mol/L) dans la solution de conversion ionique, et
$z_i$ est le nombre de charges de l'ion i et la somme porte sur les ions du ou des sels ;

f. la combinaison d'au moins une partie de la première phase non aqueuse avec la solution de conversion ionique dans un rapport entre la solution de conversion et la phase non aqueuse dans une plage de 0,1 et jusqu'à 10,0 en volume, respectivement, de préférence de 0,3 et jusqu'à 3 en volume, et plus préférablement de 0,5 et jusqu'à 1, pour former une seconde solution combinée ;
g. la fourniture de la seconde solution combinée avec une température de réaction d'au moins 130 °C, de préférence d'au moins 150 °C, plus préférablement d'au moins 170 °C, et le plus préférablement d'au moins 200 °C pour former une seconde solution combinée chauffée pour convertir au moins une partie du xylose-ester de diboronate en furfural, dans lequel la seconde solution combinée chauffée comprend une seconde phase aqueuse comprenant de l'eau et au moins 90 % des ions de la seconde solution combinée, et une seconde phase non aqueuse comprenant plus de 50 % du solvant non hydrosoluble, plus de 50 % de l'acide boronique non hydrosoluble, et au moins 50 % du furfural dans la seconde solution combinée ;
h. la séparation d'au moins une partie de la seconde phase non aqueuse de la seconde solution combinée ; et
i. la récupération d'au moins une partie du furfural de la seconde phase non aqueuse.

**2.** Procédé selon l'une quelconque revendication précédente, dans lequel le sel est un sel organique, ou un sel inorganique, ou une combinaison de ceux-ci, dans lequel le sel est hydrosoluble et insoluble dans le solvant à une température dans une plage de 20 °C à 200 °C.

**3.** Procédé selon la revendication 3, dans lequel la combinaison de sel et d'acide est choisie dans le groupe constitué de (i) $Na_2SO_4$ ou $MgSO_4$ avec $H_2SO_4$, et (ii) NaCl ou $MgCl_2$ avec HCl.

**4.** Procédé selon l'une quelconque revendication précédente, dans lequel le pH de la solution de conversion est fourni en utilisant au moins un acide choisi dans le groupe constitué d'un acide organique, d'un acide inorganique, et d'une combinaison de ceux-ci.

**5.** Procédé selon la revendication 4, dans lequel l'acide est un acide inorganique choisi dans le groupe constitué d'HCl, $H_2SO_4$, $H_3PO_4$, et une combinaison quelconque de ceux-ci.

**6.** Procédé selon la revendication 5, dans lequel au moins une partie de la première phase aqueuse en (c) comprend un solvant non hydrosoluble, et un acide boronique non hydrosoluble, ledit procédé comprenant en outre :
le traitement ultérieur d'au moins une partie de la première phase aqueuse séparée pour récupérer au moins une partie du solvant non hydrosoluble, ou de l'acide boronique non hydrosoluble, ou d'une combinaison de ceux-ci.

**7.** Procédé selon l'une quelconque revendication précédente, dans lequel l'étape (g) de fourniture de la seconde solution combinée avec la température de réaction comprend :

le chauffage de la première phase non aqueuse à la température de réaction ;
le chauffage de la solution de conversion ionique à la température de réaction ; et
la combinaison de la première phase non aqueuse chauffée avec la solution de conversion ionique chauffée pour former la seconde solution combinée chauffée.

**8.** Procédé selon l'une quelconque revendication précédente, comprenant en outre la réalisation d'au moins une partie des étapes (c) et (d) dans une unité d'extraction liquide-liquide en fonctionnement à contre-courant, dans lequel la solution contenant du xylose est fournie à une température plus élevée que la température de la solution d'extraction.

**9.** Procédé selon l'une quelconque revendication précédente, dans lequel l'acide boronique non hydrosoluble présente une hydrosolubilité à 20 °C jusqu'à 5 % en poids.

**10.** Procédé selon l'une quelconque revendication précédente, dans lequel l'acide boronique non hydrosoluble est choisi dans le groupe constitué d'acide phénylboronique, acide 4-biphénylboronique, acide 4-butylphénylboronique, acide 4-tert-butylphénylboronique, acide 4-éthylphénylboronique, acide 2-naphtylboronique, acide naphtalène-1-boronique, acide o-tolylboronique, acide m-tolylboronique, acide (2-méthylpropyl)boronique, acide butylboronique, acide octylboronique, acide phénéthylboronique, acide cyclohexylboronique, et une combinaison quelconque de ceux-ci.

**11.** Procédé selon l'une quelconque revendication précédente, dans lequel le solvant non hydrosoluble présente une hydrosolubilité à 20 °C jusqu'à 5 % en poids.

**12.** Procédé selon l'une quelconque revendication précédente, dans lequel le solvant non hydrosoluble est choisi dans le groupe constitué d'acide benzoïque, crésol (m), éther di-isopropylique, acide téréphtalique, éther diéthylique du diéthylène glycol, anisole, acide salicylique, 2,6 xylénol, 4Et-phénol, toluène, benzofurane, éthylbenzène, acide octanoïque, 1-méthylnaphtalène, nitrobenzène, gaïacol, heptane, 1-octanol et méthylisobutylcétones, et une combinaison quelconque de ceux-ci.

**13.** Procédé selon l'une quelconque revendication précédente, dans lequel au moins l'un de l'acide boronique non hydrosoluble et du solvant non hydrosoluble a un point d'ébullition supérieur à celui du furfural, de préférence supérieur d'au moins 2 °C.

**14.** Procédé selon l'une quelconque des revendications 10, 12 et 13, dans lequel l'étape (i) comprend la fourniture d'au moins une partie de la seconde phase non aqueuse de (h) à un procédé de distillation pour récupérer un produit de tête comprenant du furfural et un produit de fond comprenant un solvant non hydrosoluble et un acide boronique non hydrosoluble.

**15.** Procédé selon la revendication 14, comprenant en outre la fourniture d'au moins une partie du produit de fond pour l'utilisation en tant que partie de la solution d'extraction.

**FIG. 1**

**FIG. 2**

FIG. 3

FIG. 4

**EP 4 263 676 B1**

**Patent documents cited in the description**

- US 10407453 B
- WO 2015066287 A1
- WO 2016025678 A
- WO 2016025679 A
- US 9290821 B

**Non-patent literature cited in the description**

- **J.-P. LANGE ; E. VAN DER HEIDE ; J. VAN BUIJTENEN ; R.J. PRICE.** *ChemSusChem,* 2012, vol. 5, 150-166
- **B. R. CAES ; R. T. RAINES.** *ChemSusChem,* 2011, vol. 4, 353-356
- **L. SHUAI ; J. LUTERBACHER.** *ChemSusChem,* 2016, vol. 9, 133-155
- **STEINBACH ; KRUSE ; SAUER.** *Biomass Conv. Bioref.,* 2017, vol. 7, 247-274